# NOUVEAU FASCICULE DE BREVET EUROPEEN

(11) **EP 0 684 308 B2**
(45) Date de publication et mention de la décision concernant l'opposition: **14.01.2004**
(45) Mention de la délivrance du brevet: 15.12.1999
(21) Numéro de dépôt: 94810306.4
(22) Date de dépôt: 27.05.1994
(51) Int. Cl.: C12N 1/20, C12N 1/18, A21D 8/04, C12M 1/36, C12P 7/06

(54) **Procédé d'obtention d'une biomasse et ferment de panification**
Verfahren zur Gewinnung einer Biomasse und Brottreibmittel
Process for production of a biomass and bread leavening agent

(43) Date de publication de la demande: 29.11.1995
(73) Titulaire: AGRANO AG, CH-4123 Allschwil (CH)
(72) Inventeur: Ehret, Aloyse, F-68730 Blotzheim (FR)
(74) Mandataire: Rottmann, Maximilian R.

(56) Documents cités:
- EP-A- 0 093 635
- EP-A- 0 153 057
- EP-A- 0 229 979
- EP-A- 0 295 358
- WO-A-91/04669
- WO-A-92/20777
- BE-A- 816 571
- FR-A- 2 353 235
- GB-A- 372 738
- GB-A- 1 035 552
- US-A- 3 868 307
- DATABASE WPI Section Ch, Week 8822, Derwent Publications Ltd., London, GB; Class D16, AN 88-153243 & SU-A-1 346 676 (KAUN POLY) 23 Octobre 1987
- J.ADRIAN, G. LEGRAND, R. FRANGNE: "Dictionnaire de Biochimie Alimentaire et de Nutrition p.79" , 1981, TECHNIQUE ET DOCUMENTATION , PARIS

## Description

L'invention a trait
- à un procédé d'obtention d'une biomasse constituée de levure et de bactéries lactiques, tel que défini dans les revendications 1 à 15; et
- à un ferment de panification, tel que défini dans la revendication 16.

Il existe actuellement sur le marché de la boulangerie deux grandes catégories de produits:
1. Les produits issus de fabrication artisanale basés sur l'utilisation de levains naturels de composition indéfinie constitués d'espèces microbiologiques non identifiées eucaryotes et procaryotes dont les représentants majoritaires sont des microorganismes de type levure et/ou de type bactérie. Les espèces les plus souvent représentées sont pour les levures *Saccharomyces cerevisiae* ou *Candida,* pour les bactéries les bactéries lactiques du genre *Lactobacillus.* Ces levains représentent des mélanges de microorganismes en équilibre instable variant en fonction de paramètres propres au microcosme dans lequel ils se développent. Ces paramètres non contrôlables, tels que la nature du milieu de panification (origine des farines, nature des farines), ou paramètres physico-chimiques de mise en oeuvre (température, climat) influent sur la reproductibilité des qualités des produits finis et sur l'impossibilité de répondre à une demande d'industrialisation de la fabrication. Il faut cependant remarquer que ces levains conduisent à des produits aux qualités gustatives et organoleptiques unanimement reconnus et recherchés.
2. Les produits standardisés obtenus dans des conditions décrites dans divers brevets (p. ex. FR-A-2.525.628 (demande de brevet 82.07047), EP-B1-0.093.635), à savoir productions de microorganismes sélectionnés du type levures ou bactéries en culture pure. Ces microorganismes sont sélectionnés sur la base des particularités de leur métabolisme. A ces microorganismes sont souvent ajoutés des additifs du type ''améliorants'', permettant la mise en oeuvre rapide, la conservation, l'aspect et/ou la texture des produits finis. Ces produits sont souvent ajoutés à la levure de boulangerie traditionnelle sélectionnée et élevée industriellement sur des milieux de culture à base de mélasse sucrière et d'additifs chimiques destinés à améliorer la croissance (acide phosphorique, acide sulfurique, ammoniaque etc.).

Cette façon de procéder peut globalement se traduire par la démarche suivante: associer sous forme de poudre ou de pâte des cultures de levure de boulangeries industrielles aux cultures de microorganismes sélectionnés (levures et bactéries lactiques). Ce mélange est additionné d'additifs chimiques. La panification à partir de ces levains "reconstitués", si elle améliore nettement les qualités des produits finis par rapport aux produits de panification obtenus industriellement à partir de la seule levure de boulangerie, n'est pas satisfaisante par comparaison aux produits artisanaux, ni du point de vue des qualités organoleptiques, ni du point de vue de l'évolution de la qualité intrinsèque du produit qui met en oeuvre des additifs chimiques, enlevant ainsi la notion du "tout biologique" actuellement recherché.

De plus, la publication EP-A1 -0.093.635 décrit une composition fermentée pour la préparation d'un levain de panification et un procédé permettant la préparation d'un tel levain par culture de souches sur un milieu céréalier. Le milieu utilisé comprend de l'eau, de la farine, du germe de blé, de l'extrait de levure, du chlorure d'ammonium, du dihydrogénate de potassium, de l'antimousse, de l'alpha amylase et de l'amyloglucosidase. Le procédé de production n'envisage cependant pas l'utilisation de protéases. De plus, la réaction amyloglycolytique est effectué à pH 4.5, ce qui nécessite l'ajout d'acide et donc un contrôle du pH. La composition ainsi que le procédé de sa production ne sont donc pas "tout biologique".

Finalement, la publication WO-A1-92/20.777 décrit un procédé visant à produire de l'éthanol à partir de matières premières contenant des sucres fermentescibles ou des constituants pouvant être convertis en sucres fermentescibles. Le dit procédé comprend les étapes suivantes: (a) liquéfaction des matières premières en présence d'une α-amylase afin d'obtenir une liquéfaction de l'amidon (empois); (b) saccharification de l'empois en présence de glucoamylase pour obtenir les sucres fermentescibles; (c) fermentation des sucres par la levure afin d'obtenir de l'éthanol; et (d) séparation de l'éthanol formé, une protéase fongique étant introduite dans l'empois d'amidon pendant la phase de saccharification et/ou à l'amidon hydrolysé durant la fermentation.

Le but du procédé de cette référence n'est donc pas de produire un milieu de culture qui peut être utilisé pour la culture individuelle de levure et de bactéries lactiques ainsi que pour la coculture de levure et de bactéries lactiques mais la production d'éthanol.

Or, le but de la présente invention est de remédier à ces inconvénients en créant un procédé d'obtention d'une biomasse constituée de levure et de bactéries lactiques, sur un milieu céréalier, le produit obtenu étant directement utilisable comme ferment de panification sans séparation préalable entre milieu de culture et biomasse.

Ce but est atteint par les mesures définies dans la partie caractérisante de la revendication 1.

Le procédé selon l'invention permet d'obtenir des mélanges définis de microorganismes identifiés et sélectionnés pour leurs particularités métaboliques, notamment dans le métabolisme des sucres.

Les produits obtenus sont des mélanges définis, permettant, suivant la nature des souches sélectionnées et de leur représentativité dans les mélanges, d'obtenir en panification directe de type industriel des produits comparables à ceux obtenus avec les levains de type traditionnel, et ceci de façon parfaitement reproductible. Ces mélanges peuvent également être utilisés en panification artisanale avec les mêmes bénéfices.

L'invention fait état de culture mixte ou séquentielle. Le fait de pouvoir différer les ensemencements l'un ou les uns par rapport aux autres apportent des résultats qui ne sont pas comparables à ceux de la publication susmentionnée EP-A1-0.093.635, puisque la présente invention est capable de piloter le nombre relatif d'un microorganisme par rapport à un second voir à un troisième, ou plus, séquence d'ensemencement. Ceci se traduit par des interactions métaboliques entre les microorganismes qui sont contrôlées et qui influent sur les qualités des divers levains obtenues dans les diverses conditions de culture.

La culture est préférablement de type discontinu alimenté, dit "en fed-batch". Cette technique consiste à additionner une charge de milieu périodiquement en continu avec les ingrédients nécessaires. Ce type de fermentation permet de contrôler le métabolisme de la culture entre voie aérobie et voie anaérobie, contrôlant ainsi les flux métaboliques entre croissance (biomasse) et les produits dérivés de son métabolisme (en particulier éthanol).

On peut contrôler la teneur en éthanol dans la culture par régulation de la vitesse d'alimentation en milieu de culture, et on peut contrôler la pression partielle de l'oxygène dissout dans la culture par régulation de l'apport d'air suivant une pente préalablement définie, maintenant la culture dans un métabolisme limité en oxygène.

La culture se fait sans régulation de pH.

De préférence, la coculture se fait en une culture mixte séquentielle; c'est à dire qu'on ajoute des microorganismes différés dans le temps, permettant de réutiliser à un temps donné des produits du métabolisme des microorganismes présents précédemment. Préférablement, on contrôle le processus de culture de façon à ce que la teneur en éthanol varie entre 0,5 et 10 gramme/litre de culture.

De préférence, le procédé selon l'invention est exécuté de la façon suivante:
- on introduit un premier milieu de culture (dit "milieu de base") dans un bioréacteur;
- on ensemence ce premier milieu de culture avec la levure et les bactéries lactiques à cultiver distribués dans un deuxième milieu de culture (dit "milieu de dosage");
- on alimente le bioréacteur en continu avec le deuxième milieu de culture;
tout en maintenant une température d'environ 30 °C, une aération contrôlée par la pression partielle d'oxygène, et une concentration en éthanol entre 0,5 et 10 gramme/litre de culture.

Le produit obtenu peut être utilisé, dès sa sortie du bioréacteur, comme ferment en panification directe industrielle sans opération ultérieure, à savoir sans séparation préalable entre milieu de culture et biomasse. Cependant, en règle générale il est préférable de concentrer le produit par centrifugation ou par filtration, puisque alors le produit réduit en sa teneur d'eau peut être conservé à 3 °C pendant 21 jours sans perte notable de son pouvoir levant.

De préférence, la levure mise en oeuvre est une souche de *Saccharomyces cerevisiae,* préférablement une souche isolée d'un levain naturel, et en particulier la souche *Saccharomyces cerevisiae steineri* DSM 9211. Cette souche a été isolée à partir d'un levain artisanal d'excellente qualité organoleptique. Les caractéristiques du genre *Saccharomyces cerevisiae* sont compilés dans le Tableau 1.

**Tableau 1**

| Caractéristiques de *Saccharomyces cerevisiae* | | |
|---|---|---|
| Culture en milieu liquide (milieu de dosage dilué au ½): | | |
| Observation au microscope: Les cellules de levure sont ovales (2 à 4 x 5 à 7 micromètre) et se divisent par bourgeonnement multipolaire. | | |
| Culture en milieu solide (milieu de dosage dilué au ½ additionné de 15 gramme/litre de gélose) : | | |
| Observation des colonies: Les colonies de levure sont rondes, lisses, mates, légèrement bombées et de couleur crème. | | |
| Métabolisme des sucres: | | |

| | Assimilation | Fermentation |
|---|---|---|
| Glucose | + | + |
| L-Arabinose | - | - |
| D-Xylose | - | - |
| Galactose | + | + |
| Cellobiose | - | - |
| Lactose | - | - |
| Maltose | + | + |
| Saccharose | + | + |
| Tréhalose | - | - |
| Mélézitose | - | - |
| Raffinose | + | - |

De préférence, la bactérie lactique mise en oeuvre est des genres *Lactobacillus, Leuconostoc* et/ou *Pediococcus*, préférablement une souche de *Lactobacillus brevis, Lactobacillus plantarum, Leuconostoc mesenteroides* et/ou *Pediococcus pentosaceus,* et en particulier une ou plusieurs des souches *Lactobacillus brevis* DSM 9209, *Lactobacillus plantarum* DSM 9208, *Leuconostoc mesenteroides* DSM 9207 et *Pediococcus pentosaceus* DSM 9210. Ces souches ont été isolées à partir du même levain qui était la base pour l'isolement de la souche *Saccharomyces cerevisiae steineri* DSM 9211 mentionnée ci-dessus. Les caractéristiques des dites souches sont compilées dans le Tableau 2.

**Tableau 2**

| Caractéristiques des souches de bactéries lactiques | | | | |
|---|---|---|---|---|
| | *L. plantarum* DSM 9208 | *L. brevis* DSM 9209 | *L. mesenteroides* DSM 9207 | *P. pentosaceus* DSM 9210 |
| Contrôle | - | - | - | - |
| | | | | |
| G lycérol | - | - | - | - |
| Érythritol | - | - | - | - |
| D-Arabinose | - | - | - | - |
| L-Arabinose | + | + | + | + |
| Ribose | + | + | + | + |
| | | | | |
| D-Xylose | - | + | + | - |
| L-Xylose | - | - | - | - |
| Adonitol | - | - | - | - |
| beta-Methyl-xyloside | - | + | - | - |
| Galactose | + | + | + | + |
| | | | | |
| D-Glucose | + | + | + | + |
| D-Fructose | + | +/- | + | + |
| D-Mannose | + | - | + | + |
| L-Sorbose | - | - | - | - |
| Rhamnose | +/- | - | - | - |
| | | | | |
| Dulcitol | - | - | - | - |
| I nositol | - | - | - | - |
| Mannitol | + | - | - | - |
| Sorbitol | + | - | - | - |
| alpha-Methyl-D-mannoside | + | - | - | - |
| alpha-Methyl-D-glucoside | + | + | + | - |
| N-Acetyl- | + | +/- | + | + |
| glucosamine | | | | |
| Amygdaline | + | - | + | - |
| Arbutine | + | - | + | + |
| Esculine | + | - | + | + |
| | | | | |
| Salicine | + | - | + | + |
| Cellobiose | + | - | + | + |
| Maltose | + | + | + | + |
| Lactose | + | - | - | - |
| Mélibiose | + | +/- | + | - |
| | | | | |
| Saccharose | + | - | + | - |
| Tréhalose | + | - | + | - |
| Inuline | - | - | - | - |
| Mélézitose | + | - | - | - |
| D-Raffinose | + | - | + | - |
| | | | | |
| Amidon | - | - | - | - |
| Glycogène | - | - | - | - |
| Xylitol | - | - | - | - |
| beta-Gentibiose | +/- | - | +/- | + |
| D-Turanose | + | - | + | - |
| D-Lyxose | - | - | - | - |
| D-Tagatose | - | - | - | + |
| D-Fucose | - | - | - | - |
| L-Fucose | - | - | - | - |
| D-Arabilol | +/- | - | - | - |
| | | | | |
| L-Arabitol | - | - | - | - |
| Gluconate | +/- | +/- | - | - |
| 2-Céto-gluconate | - | - | - | - |
| 5-Céto-gluconate | - | +/- | - | - |

De préférence, on fait croître dans un bioréacteur dans un système mixte et/ou séquentiel la souche de levure en culture discontinue alimentée avec une ou plusieurs souches bactériennes suivant la nature du produit de panification désirant être obtenu en final.

Le procédé selon l'invention permet un contrôle du métabolisme de chaque microorganisme, permettant d'agir sur la concentration des métabolites terminaux, en particulier l'éthanol, l'acide lactique et l'acide acétique. Le choix des microorganismes basé sur les particularités métaboliques, en particulier les voies métaboliques homofermentaires ou hétérofermentaires, et notamment la régulation de l'activité enzymatique pyruvate oxydase oxygène dépendante (voir: Frey, Le Lait 1992) permet de contrôler d'une manière fine la concentration de l'acide acétique dans le mélange final.

D'autre part, le procédé selon l'invention permet de différer dans le temps (système séquentiel) la mise en oeuvre d'un microorganisme par rapport à un autre.

Plusieurs possibilités sont offertes.
1) La mise en culture simultanée d'une ou plusieurs levures et d'une ou plusieurs bactéries lactiques (culture mixte).
2) La mise en culture d'une ou plusieurs levures pendant une période définie, puis mise en culture dans la culture de levure ou des levures d'une ou plusieurs bactéries lactiques (culture séquentielle).

La mise en culture des bactéries lactiques peut elle-même se faire simultanément (plusieurs souches ensemencées en même temps) ou de façon différée (une première souche mise en coculture avec la levure ou les levures au temps t₁, puis une deuxième souche mise en culture au temps t₂...tₓ).

De la même façon, la culture des bactéries lactiques peut précéder la mise en coculture de la levure ou des levures.

Le Tableau 3 résume ces possibilités sous forme d'un schéma simplifié.

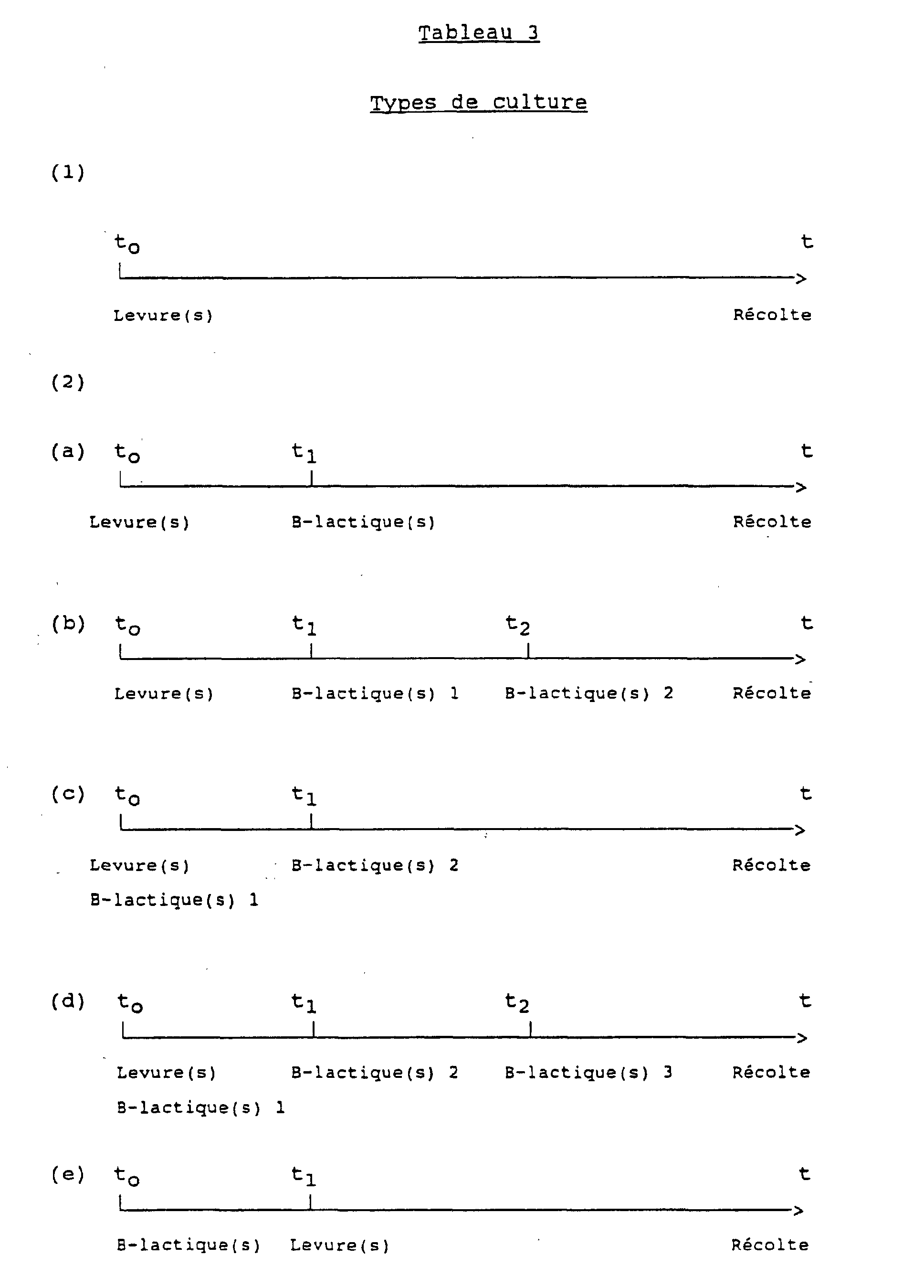

### Exemples

### 1. Ingrédients utilisés pour la préparation des milieux de culture

Les ingrédients suivants sont utilisés pour la préparation des milieux de culture décrits ci-après:
- Grains de blé, moulus extemporanément avant utilisation pour garder l'intégralité des éléments nutritifs. Une analyse type du produit est la suivante:
   - eau environ 13%,
   - protéines totales environ 12%,
   - hydrates de carbone environ 69 %,
   - lipides totaux environ 2 %,
   - amidon environ 59 %,
   - cendres environ 1,5 %.
- Germes de blé, moulu à vitesse réduite avec échauffement réduit contrôlé. Un taux de lipides entre 11 et 12 % et un taux d'amidon inférieur à 10 % sont les critères retenus pour confirmer la qualité du produit. Une analyse type du produit est la suivante:
   - eau environ 13 %,
   - protéines totales environ 31,5 %,
   - hydrates de carbone environ 25 %,
   - lipides totaux environ 11 %,
   - amidon environ 8 %,
   - cendres environ 5 %.
- Autolysat de levure de qualité alimentaire apportant des vitamines et des acides aminés au milieu. Une analyse type du produit est la suivante:
   - eau environ 3,5 %,
   - protéines totales environ 50,5 %,
   - hydrates de carbone environ 32 %,
   - lipides totaux environ 5 %,
   - amidon environ 1,5 %,
   - cendres environ 7,5 %.
- Sel marin
- Eau industrielle.

### 2. Préparation du milieu dit "milieu de dosage"

Dans un bioréacteur de 15 litres, on mélange 8 litres d'eau, 1660 grammes de grains de blé moulus, 1000 grammes de germe de blé, 100 grammes d'autolysat de levure, 30 grammes de sel de mer, 1 ml d'une solution d'alpha-amylase (16 unités RAU/gramme d'amidon à hydrolyser).

Le mélange est porté à 85 °C pendant 20 minutes puis refroidit à 75 °C. Ensuite 2 ml du même enzyme sont ajoutés. La température est maintenue pendant 20 minutes.

Le mélange est refroidit à 60 °C. On ajoute une solution d'amyloglucosidase à raison de 50 ml. L'action de l'enzyme est poursuivie pendant 90 minutes. Le mélange est refroidit à 50 °C et soumis à l'hydrolyse par deux protéases spécifiques, la première étant de la papaïne purifiée et fractionnée, et la deuxième étant de la pancréatine fractionnée.

On utilise 1,5 ml de la première protéase par kilogramme de farine et 2,3 grammes de la deuxième protéase par kilogramme de farine. L'action des protéases dure 220 minutes.

Le milieu de culture obtenu est stérilisé à 120 °C pendant 20 minutes. Ce milieu parfaitement stable est conservé à +4 °C.

### 3. Préparation du milieu dit "milieu de base"

Ce milieu sert d'ensemencement dit en terme technique "de pied de cuve" dans le système de production de la biomasse suivant le procédé de culture discontinue alimentée ("en fed-batch").

Dans un bioréacteur on mélange 10 titres d'eau, 500 grammes de germe de blé, 70 grammes d'autolysat de levure et 30 grammes de sel marin.

Le milieu est soumis à l'hydrolyse par l'alpha-amylase (2 ml/kg de germe de blé) pendant 20 minutes à 75 °C puis à l'action de deux protéases spécifiques mentionnées ci-dessus, à savoir de la papaïne purifiée et fractionnée et de la pancréatine fractionnée, à 50 °C pendant 20 minutes. Ce milieu est conservé à +4 °C.

L'ajout d'alpha-amylase en deux temps dans le milieu dit "milieu de dosage" permet d'éviter la gélatinisation irréversible de l'amidon au moment du chauffage au-dessus de 65 °C.

Pour l'analyse, les sucres libérés sont dosés par chromatographie liquide à hautes performances, et les acides aminés libérés par l'hydrolyse sous l'action des protéases sont dosés par le réactif à la ninhydrine (S. Moore et W H. Stein, J. Biol. Chem. 176, 367, 1948).

Les valeurs moyennes obtenues sont:
- Milieu de base:
   - glucose environ 6 gramme/litre,
   - maltose environ 12,5 gramme/litre,
   - acides aminés environ 6,5 gramme/litre.
- Milieu de dosage:
   - glucose environ 86,5 gramme/litre,
   - maltose environ 11 gramme/litre,
   - acides aminés environ 9 gramme/litre.

A aucun moment dans la préparation des milieux n'entrent en jeu des additifs chimiques. Le pH final des milieux est voisin de 6,0, p. ex. 5,5 à 6,5.

### 4. Culture des microorganismes

Cet exemple décrit une culture du type (1 ) dans le Tableau 3.

Les microorganismes sont conservés au laboratoire dans les conditions traditionnelles et repiqués à deux reprises sur le milieu précédemment défini de façon à adapter les microorganismes au milieu de culture.

### - Culture de levure

La levure est conservée à -80 °C dans un milieu céréalier contenant du glycérol. Elle est réisolée sur le milieu céréalier solide. Une colonie isolée est ensemencée dans le milieu céréalier liquide (milieu de dosage dilué au ½ avec de l'eau).
Une deuxième culture à partir de la première est faite dans un Erlenmeyer de 200 ml. La densité cellulaire obtenue après 16 heures de culture à 30 °C avec agitation est de 3·10⁸ cellules/ml.
Une troisième culture à partir de la deuxième est faite dans 600 ml ensemencés par 20 ml de la culture précédente. La densité cellulaire obtenue après 8 heures de culture à 30 °C est de 2,5·10⁸ cellules/ml. Cette culture est utilisée pour ensemencer un bioréacteur de 15 litres de volume (10 litres de volume utile). La teneur en éthanol mesurée est de 25 gramme/litre. Le glucose a été entièrement métabolisé.

### - Culture des bactéries lactiques

Les souches sélectionnées pour leur métabolisme et leurs caractères organoleptiques sont conservées à -80 °C. Au moment de l'utilisation, ces souches sont repiquées sur le milieu céréalier solide.
Deux cultures sont faites successivement dans le milieu de dosage dilué au ¼ avec de l'eau.
La première culture se fait à partir d'une colonie isolée mise dans 100 ml de milieu. La densité cellulaire atteinte après 24 heures de culture à 30 °C en conditions non agitées est de 1 à 5·10⁹ cellules/ml suivant l'espèce utilisée.
La deuxième culture se fait dans un volume de 900 ml de milieu neuf ensemencé par les 100 ml de la culture précédante. Cette culture est utilisée pour ensemencer le bioréacteur à temps voulu et à la concentration désirée.

Les particularités des souches de bactéries lactiques utilisées sont compilées dans les Tableaux 4 à 6.

**Tableau 4**

| *Lactobacillus plantarum* DSM 9208 | | | | |
|---|---|---|---|---|
| 1^{re} culture: 1 oese (100 ml de milieu de dosage dilué au ½) 16 heures - 30 °C - sans agitation pH 3,8 - acidité 8,6 - numération 1,5-10⁹ c/ml | | | | |
| 2^{e} culture: dilution de la 1^{re} culture à 1/10 | | | | |

| 2^{e} culture | 0 h | 8 h | 16 h | 24 h |
|---|---|---|---|---|
| Numération [1-10⁹c/ml] | 0,15 | 1,3 | 2,50 | 3,2 |
| pH | 5,80 | 4,23 | 3,79 | 3,40 |
| Acidité | 2,50 | 4,80 | 6,50 | 9,60 |
| Glucose [g/l) | 25,9 | 20,8 | 28,5 | 16,4 |
| Acide lactique [g/l] | 0,4 | 3,2 | 5,2 | 8,1 |
| Acide acétique [g/l] | 0 | 0 | 0 | 0 |
| Éthanol [g/l] | 0 | 0 | 0 | 0 |

**Tableau 5**

| *Lactobacillus brevis* DSM 9209 | | | | |
|---|---|---|---|---|
| 1^{re} culture: 1 oese (100 ml de milieu de dosage dilué au ½) 16 heures - 30°C - sans agitation pH 4,46 - acidité 7,8 - numération 3,8·10⁹ c/ml | | | | |
| 2^{e} culture: dilution de la 1^{re} culture à 1/10 | | | | |

| 2^{e} cutture | 0 h | 8 h | 16 h | 24 h |
|---|---|---|---|---|
| Numération [1.10⁹ c/ml] | 0,30 | 1,2 | 2,0 | 3,2 |
| pH | 5,99 | 5,07 | 4,75 | 4,45 |
| Acidité | 2,0 | 2,8 | 3,5 | 4,0 |
| Glucose [g/l] | 27,2 | 25,3 | 24,1 | 22,7 |
| Acide lactique [g/l] | 0,40 | 0,84 | 1,15 | 1,65 |
| Acide acétique [g/l] | 0,20 | 0,42 | 0,50 | 0,80 |
| Éthanol [g/l] | 0,10 | 0,17 | 0,25 | 0,50 |

**Tableau 6**

| *Leuconostoc mesenteroides* DSM 9207 | |
|---|---|
| 1^{re} culture: 1 oese (100 ml de milieu de dosage dilué au ½) 16 heures - 30 °C | |
| 2^{e} culture: dilution de la 1^{re} culture à 1/10 24 heures - 30 °C - sans agitation | |

| 2ème culture | 24 h |
|---|---|
| Numération [1.10⁹ c/ml] | 1,8 |
| pH | 3,85 |
| Acidité | 9,6 |
| Glucose [g/l] | 28,3 |
| Acide lactique [g/l] | 3,02 |
| Acide acétique [g/l] | 0,57 |
| Éthanol [g/l] | 0,89 |

**Tableau 7**

| *Pediococcus pentosaceus* DSM 9210 | |
|---|---|
| 1 ^{re} culture: 1 oese (100 ml de milieu de dosage dilué au ½) 16 heures - 30 °C | |
| 2^{e} culture: dilution de la 1^{re} culture à 1/10 24 heures - 30 °C - sans agitation | |

| 2^{e} culture | 24 h |
|---|---|
| Numération [1·10⁹ c/ml] | 2,3 |
| pH | 3,42 |
| Acidité | 11,5 |
| Glucose [g/l] | 15,6 |
| Acide lactique [g/l] | 9,5 |
| Acide acétique [g/l] | 0 |
| Éthanol [g/l] | 0 |

### 5. Mise en oeuvre de la culture mixte ou séquentielle

Le but ultime qui conditionne la mise en oeuvre du procédé selon l'invention est d'obtenir une préparation constituée d'une culture mixte permettant la fabrication d'un pain comparable au pain dit de levain traditionnel. Cette croissance microbienne mixte sous-tend des interactions nombreuses de type commensalisme, coopérativité et ammensalisme entre les souches. Le procédé décrit ci-après respecte un compromis visant à produire des lactobacilles en quantité suffisante pour fournir aux pains des arômes et une acidité caractéristiques et à respecter une proportion suffisante de levure (agent levant) permettant d'obtenir sans ajout de levure externe un pain alvéolé de bonne densité.

### Exemple 1

La culture débute par une croissance de la levure. Dans un bioréacteur de 15 litres (volume utile de 10 litres), on introduit successivement 5 litres de milieu de base et 600 ml d'une culture de levure en Erlenmeyer comme décrit précédemment. Le mélange est alimenté en milieu de dosage, la température étant maintenue à 30 °C. Le pH mesuré en permanence n'est pas régulé. Il est de 6 au départ et passe à 4,0 à 5,0 en fin de culture mixte selon la souche de bactérie lactique utilisée. L'éthanol est maintenu entre 0,5 et 10 gramme/litre, par asservissement de l'alimentation en milieu de dosage.

L'agitation se fait par l'intermédiaire de deux bipales de type Rushton (taille des pales: 1/3 du diamètre du bioréacteur; coefficient de transfert: 600 mmoles O₂/litre; vitesse d'agitation: 500 à 1200 rpm; débit d'air stérile: variable entre 0 et 30 litre/minute. Le débit est conditionné par la pression partielle d'oxygène mesurée en permanence par une sonde pO₂ (Ingold) et maintenue supérieure à 30 %. L'ensemencement des bactéries lactiques se fait en différé et permet d'ajuster les concentrations des divers microorganismes et d'influencer par là les qualités organoleptiques du produit final.

Dans le Tableau 8 est représente une culture séquentielle dans laquelle *Lactobacillus plantarum* est ajouté à la culture de levure après 8 heures de croissance de celle-ci.

### Exemple 2

Cet exemple (voir Tableau 9) représente une coculture dans laquelle, au départ, il y a une culture mixte levure et *Leuconostoc mesenteroides.* Cette culture mixte est poursuivie durant 8 heures. Au bout de 8 heures est ajoutée une préculture de *Leuconostoc mesenteroides.* Le produit final est obtenu après 18 heures.

### Exemple 3

Cet exemple (voir Tableau 10) représente une coculture du même type que l'exemple 2, mais dans cet essai *Leuconostoc mesenteroides* est remplacé par *Lactobacillus brevis* dans la coculture de départ. *Lactobacillus plantarum* est ajouté après 8 heures.

On remarque dans ce cas l'augmentation de production de l'acide lactique par rapport à l'exemple 2 (2,40 gramme/litre contre 1,81 gramme/litre).

On peut également remarquer que dans le cas de coculture une partie des métabolites terminaux produits par une des espèces microbiennes est réutilisée par les autres espèces influençant les qualités organoleptiques finales (l'acide lactique réutilisé et production de l'acide acétique).

Suivant les cas (voir Tableaux 11 à 14), les densités cellulaires obtenues entre levures et bactéries sont dans un rapport de 3 à 5 (10⁹ levures pour 3 à 5·10⁹ bactéries).

La culture terminée est refroidie à 3 °C le plus rapidement possible. Elle peut être stockée pendant 8 jours sans perdre son aptitude de panification. Elle est utilisée dans un dosage à 20 % (poids/volume).

Les goûts de "levain" des pains obtenus sont excellents.

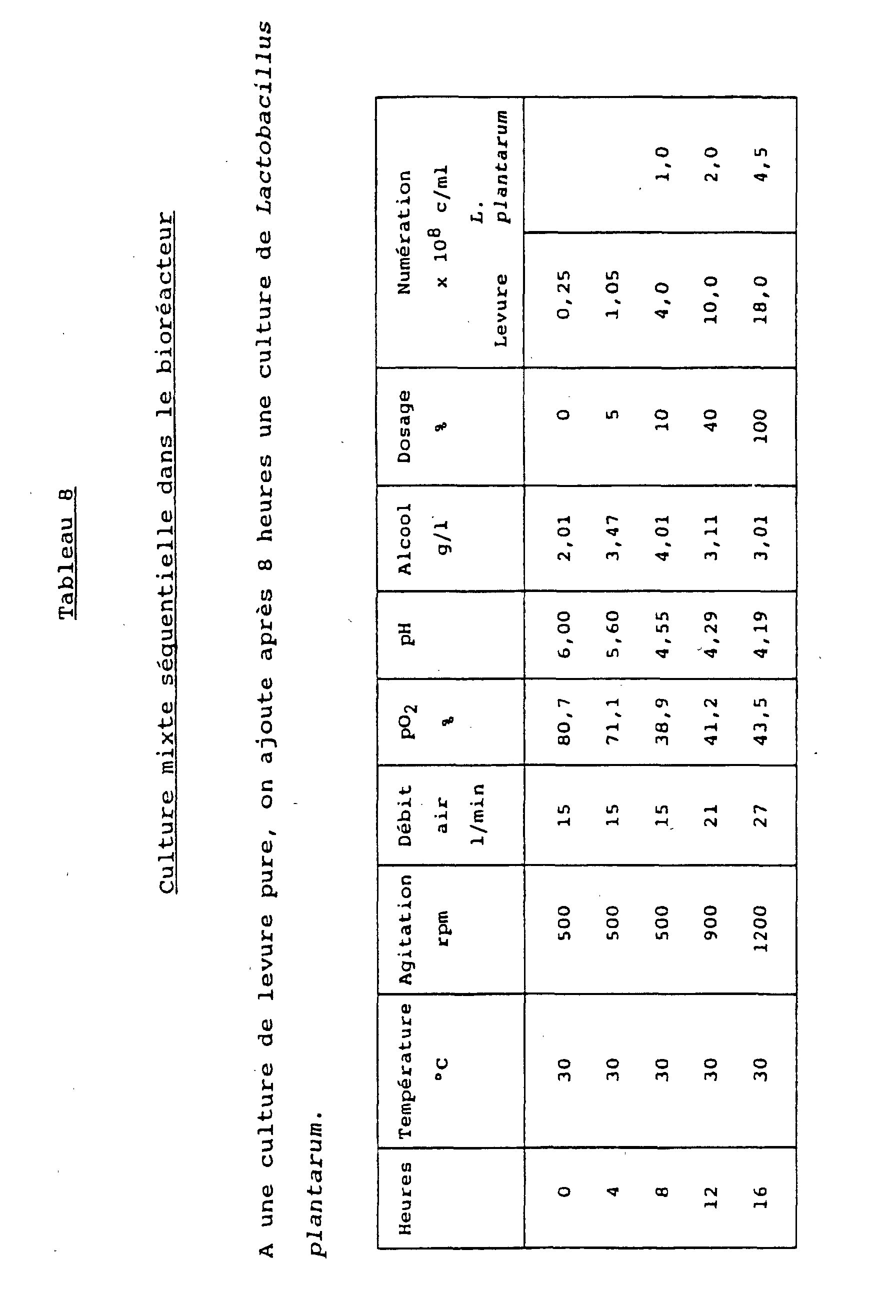

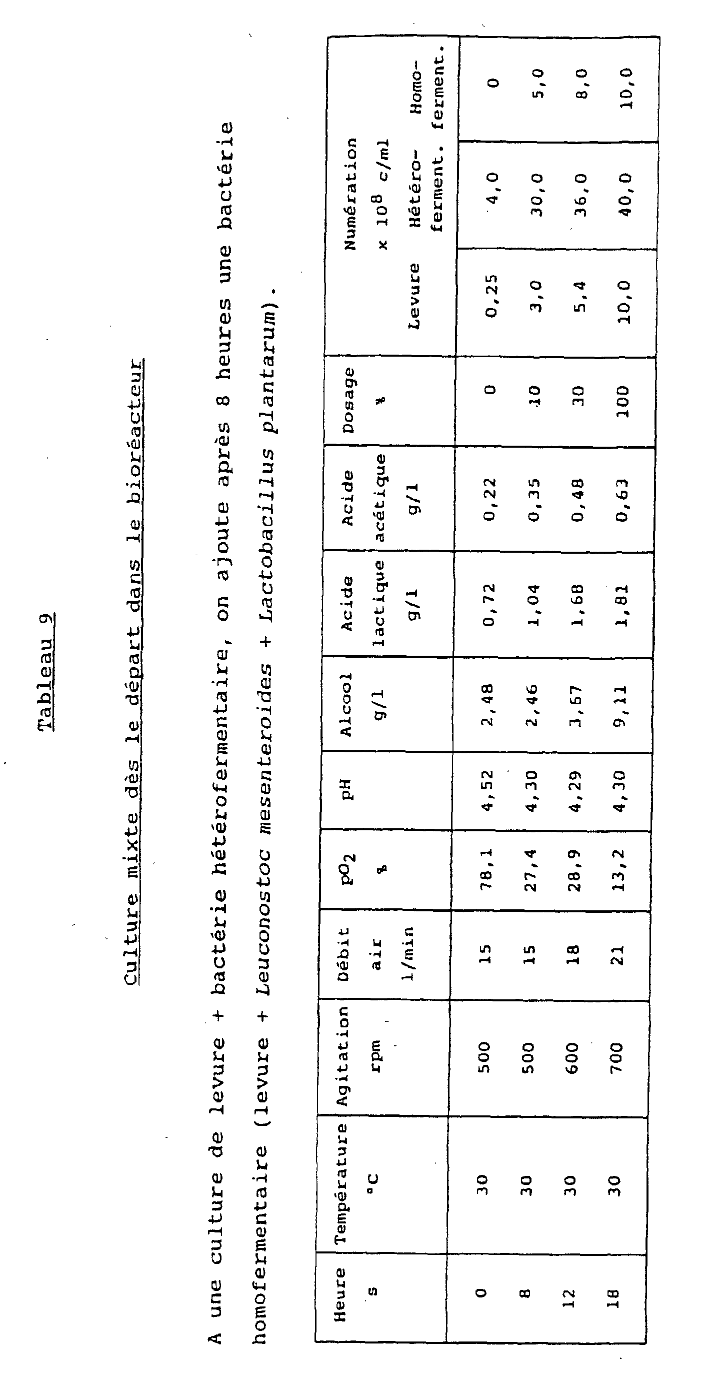

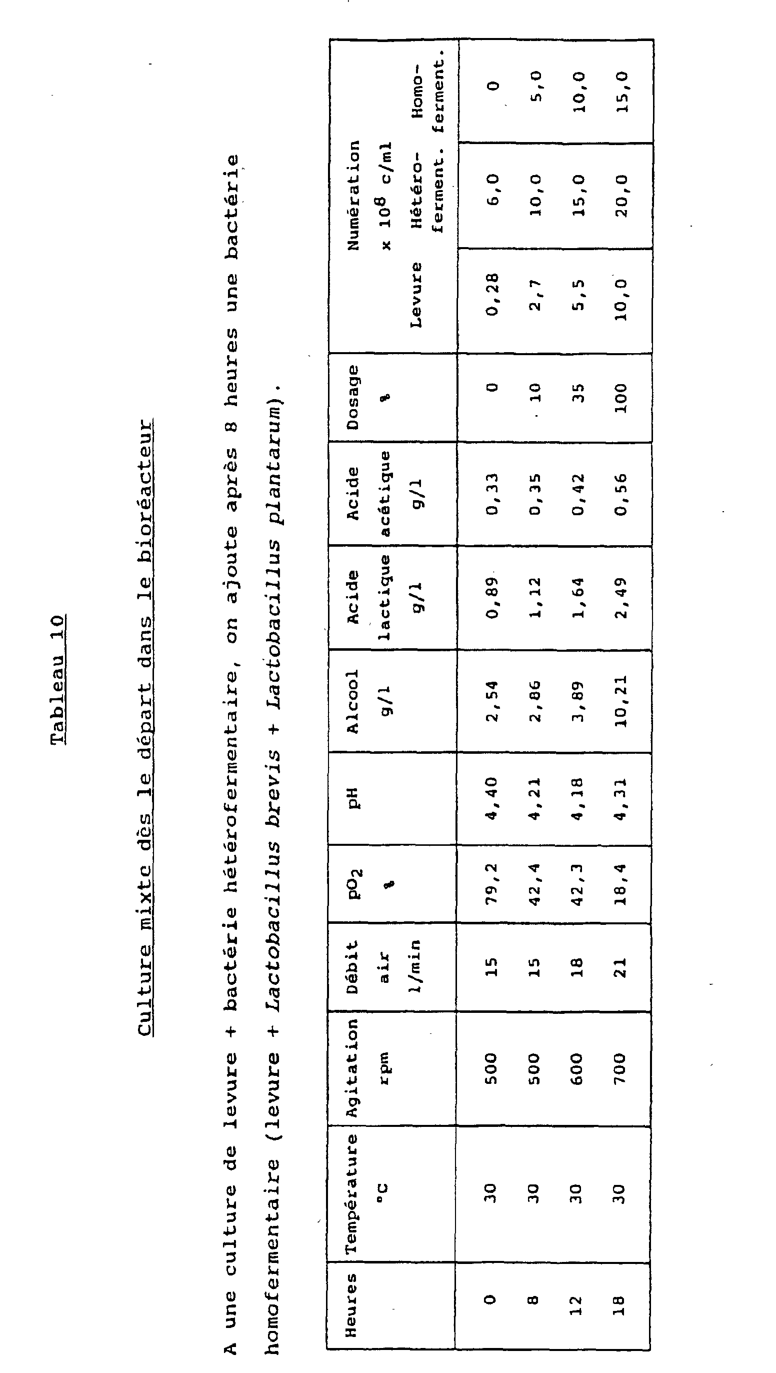

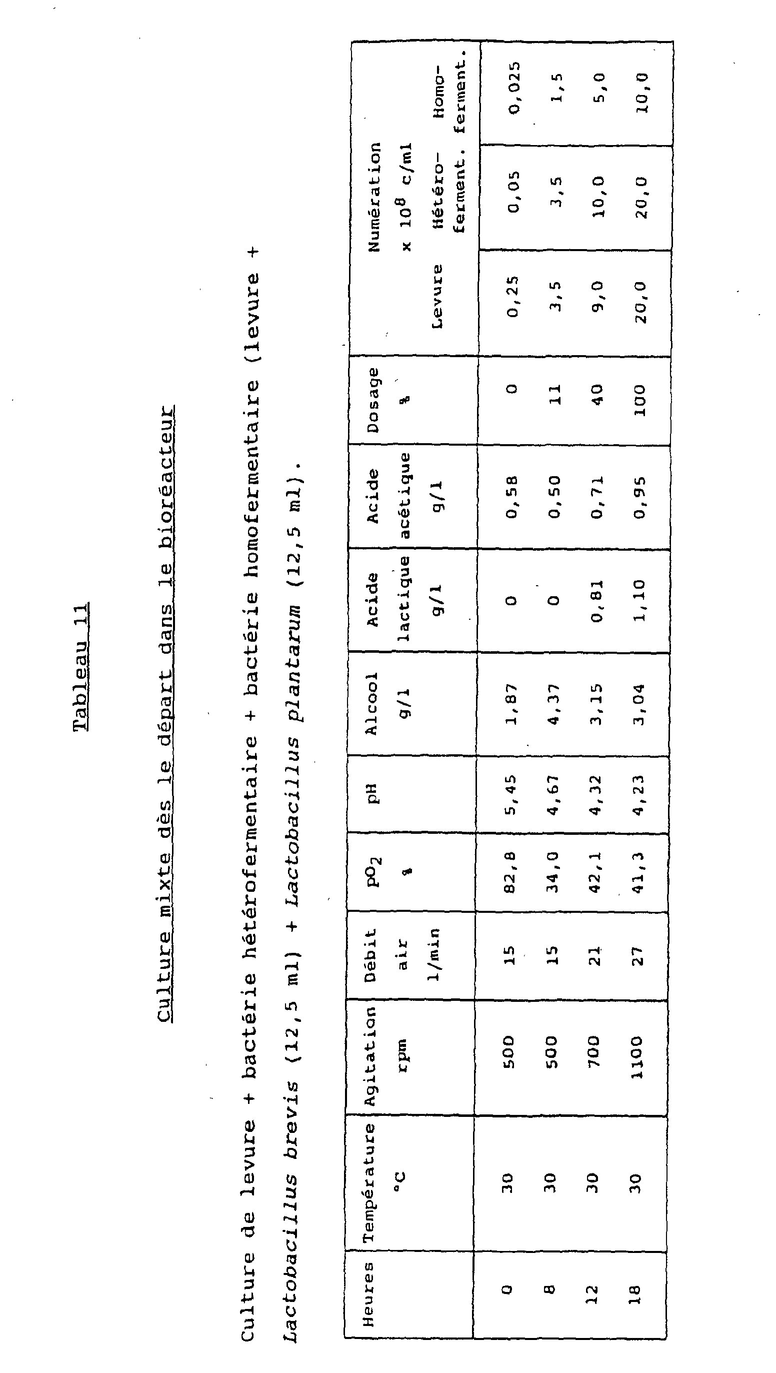

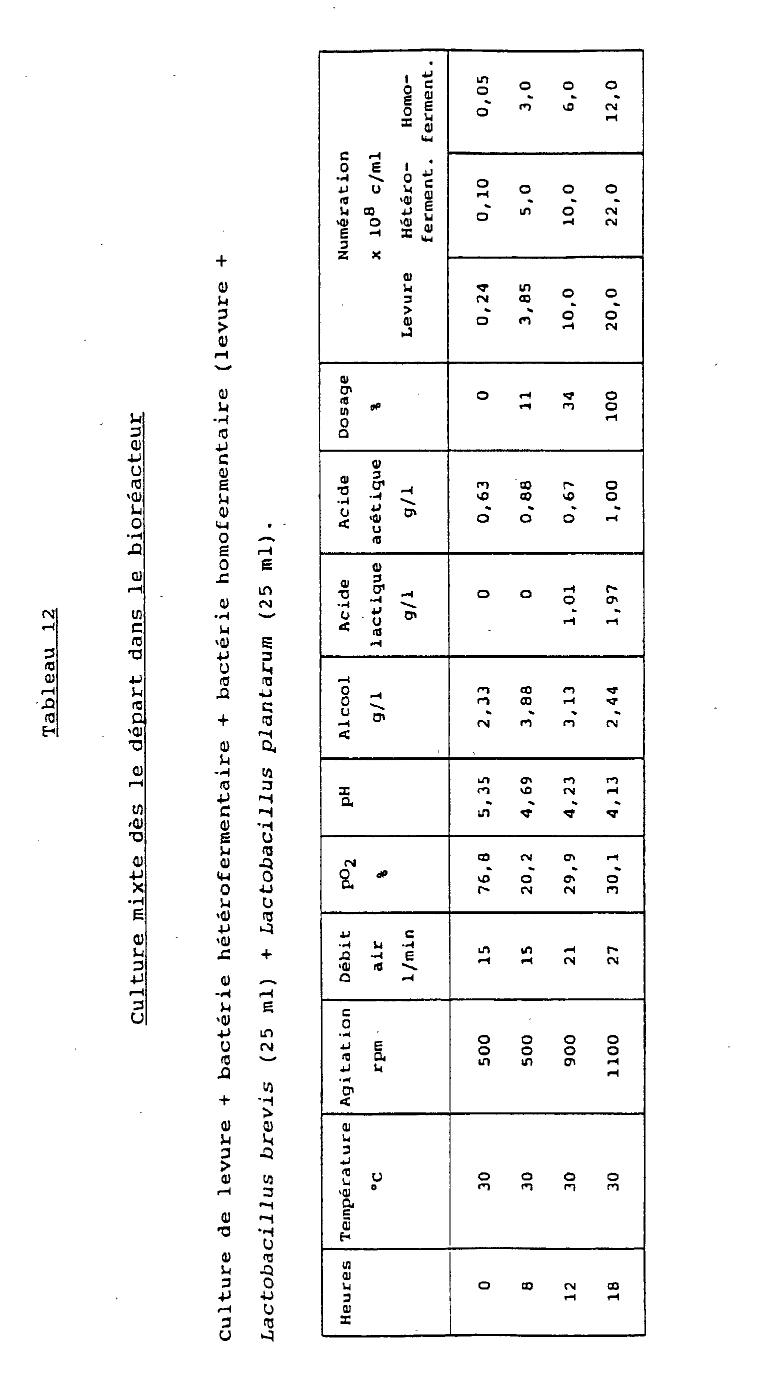

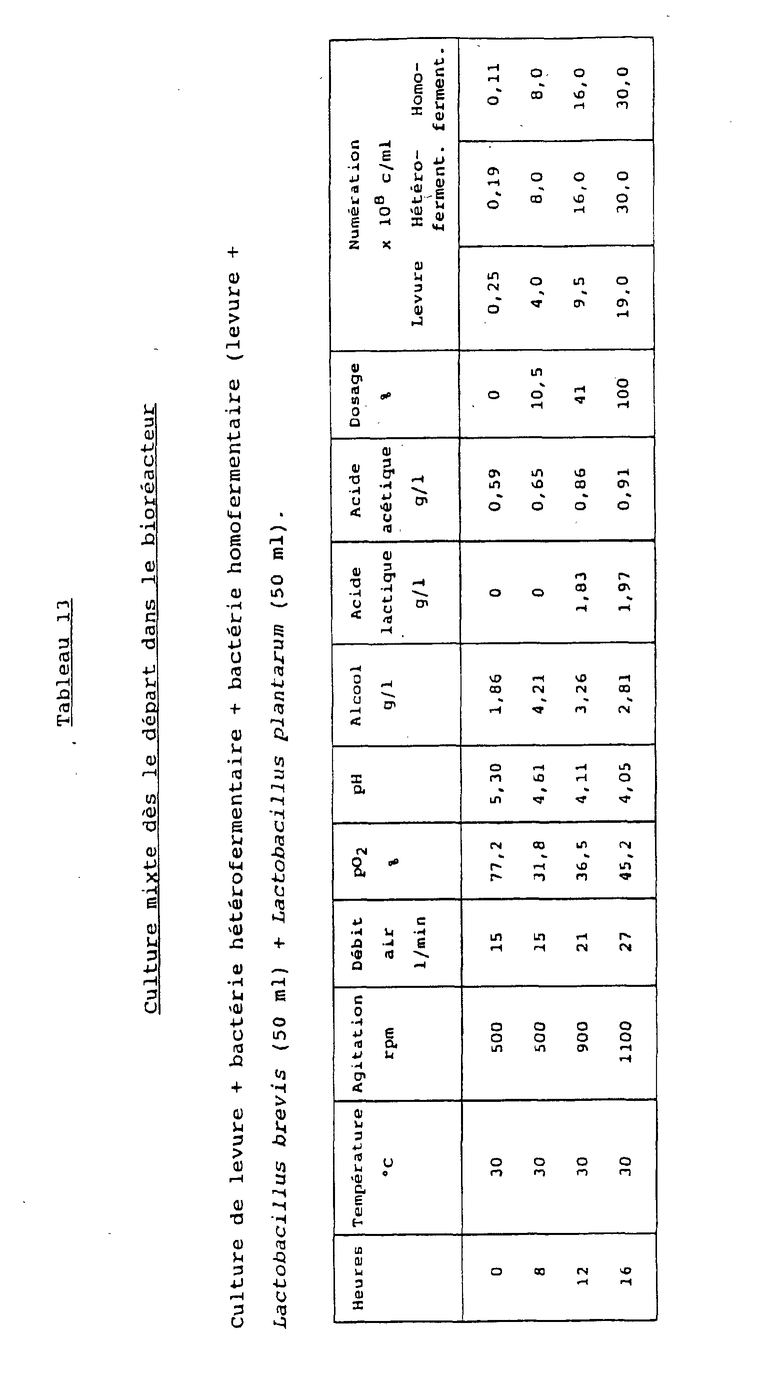

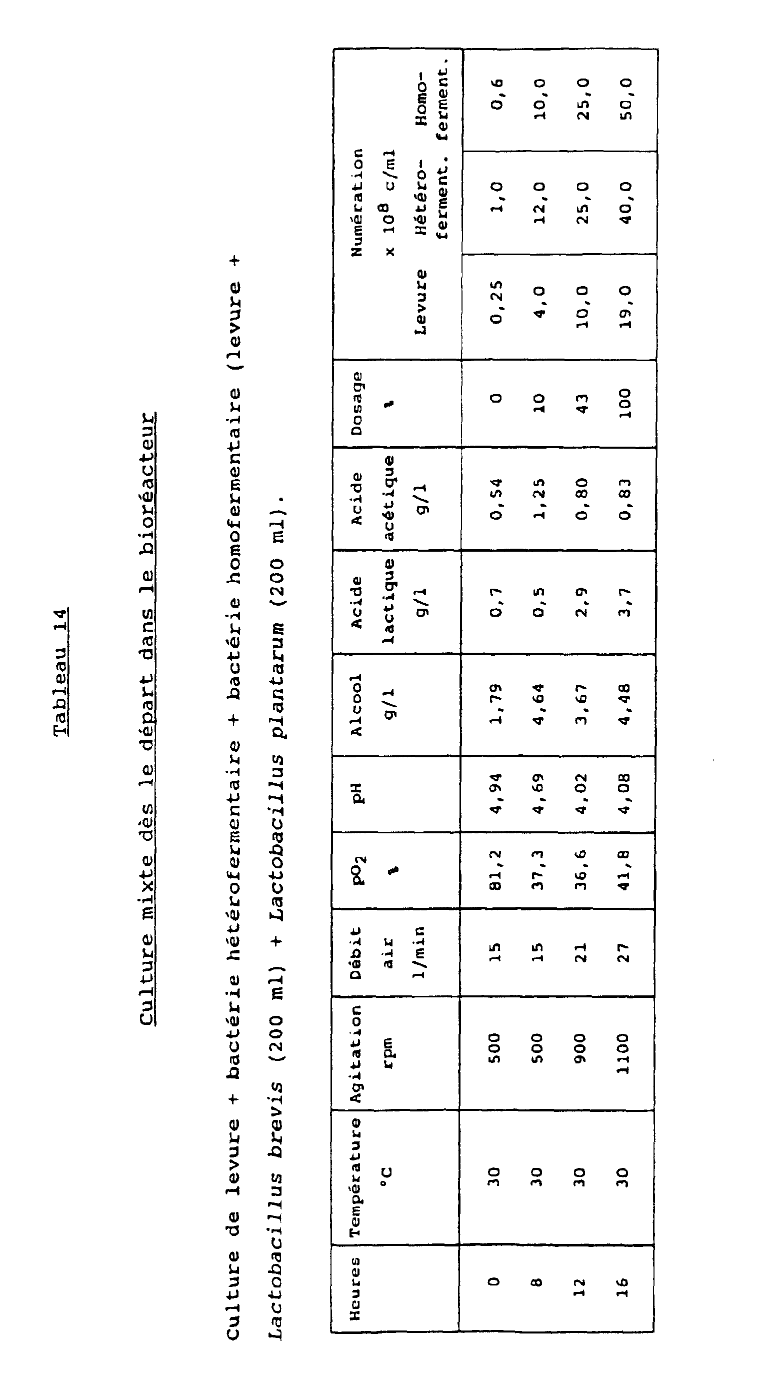

## Revendications

1. Procédé d'obtention d'une biomasse constituée de levure et de bactéries lactiques, sur un milleu céréalier, le produit étant directement utilisable comme ferment de panification sans séparation préalable entre milieu de culture et biomasse, **caractérisé en ce qu'**on cocultive sans régulation de pH au moins une souche de levure et au mains une souche de bactérie lactique en culture mixte et/ou en culture séquentielle, sur un milieu de culture obtenu:
- par une double hydrolyse d'un mélange dilué aqueux consistant en de l'eau, de la farine intégrale et/ou du germe de blé ainsi que de l'autolysat de levure et du sel marin, à savoir
- par l'hydrolyse totale de l'amidon en sucres fermentescibles par l'action d'au moins une alpha-amylase et d'au moins une amyloglucosidase, et
- par l'hydrolyse avec ménagement d'au moins une partie du gluten par des enzymes protéolytiques de qualité alimentaire; et
- ce milieu de culture étant exempt de tout additif chimique autre que les ingrédients mentionnés ci-dessus.

2. Procédé selon la revendication 1, **caractérisé en ce que** la culture est du type discontinu alimenté.

3. Procédé selon la revendication 1 ou 2, **caractérisé en ce qu'**on contrôle la teneur en éthanol dans la culture par régulation de la vitesse d'alimentation en milieu de culture.

4. Procédé selon une des revendication 1 à 3, **caractérisé en ce qu'**on contrôle la pression partielle de l'oxygène dissout dans la culture par régulation de l'apport d'air suivant une pente préalablement définie maintenant la culture dans un métabolisme limité en oxygène.

5. Procédé selon une des revendication 1 à 3, **caractérisé en ce qu'**on contrôle la pression partielle de l'oxygène dissout dans la culture par la vitesse d'agitation suivant une pente préalablement définie maintenant la culture dans un métabolisme limité en oxygène.

6. Procédé selon une des revendications 1 à 5, **caractérisé en ce que** la levure mise en oeuvre est une souche de Saccharomyces *cerevisiae.*

7. Procédé selon la revendication 6, **caractérisé en ce que** la levure mise en oeuvre est une souche de *Saccharomyces cerevisiae* isolée d'un levain naturel.

8. Procédé selon la revendication 7, **caractérisé en ce que** la levure mise en oeuvre est la souche *Saccharomyces cerevisiae steineri DSM* 9211*.*

9. Procédé selon une des revendications 1 à 8, **caractérisé en ce que** la bactérie lactique mise en oeuvre est des genres *Lactobacillus, Leuconostoc* et/ou *Pediococcus.*

10. Procédé selon la revendication 9, **caractérisé en ce que** la bactérie lactique mise en oeuvre est une souche de *Lactobacillus brevis, Lactobacillus plantarum, Leuconostoc mesenteroides* et/ou *Pediococcus pentosaceus.*

11. Procédé selon la revendication 10, **caractérisé en ce que** la bactérie lactique mise en oeuvre est une ou plusieurs des souches *Lactobacillus brevis* DSM 9209, *Lactobacillus plantarum* DSM 9208, *Leuconostoc mesenteroides* DSM 9207 et *Pediococcus pentosaceus* DSM 9210.

12. Procédé selon une des revendications 1 à 11, en ce que la coculture se fait en une culture mixte séquentielle.

13. Procédé selon la revendication 12, **caractérisé en ce que** la teneur en éthanol durant le processus de culture varie entre 0,5 et 10 gramme/litre de culture.

14. Procédé selon une des revendications 1 à 13, **caractérisé en ce qu'**on
- introduit un milieu de base dans un bioréacteur;
- ensemence ce milieu de base avec la levure et les bactéries lactiques à cultiver distribuées dans un milieu de dosage; et
- alimente le bioréacteur en continu avec le milieu de dosage
tout en maintenant une température d'environ 30 °C, une aération contrôlée par la pression partielle d'oxygène, et une concentration en éthanol entre 0,5 et 10 gramme/litre de culture.

15. Procédé selon une des revendications 1 à 14, **caractérisé en ce qu'**on concentre le produit obtenu par centrifugation ou par filtration.

16. Ferment de panification, **caractérisé en ce qu'**il est la biomasse obtenue par le procédé de la revendication 1 et qu'il comprend comme levure la souche *Saccharomyces cerevisiae steineri DSM* 9211 et comme bactérie lactique une ou plusieurs des souches *Lactobacillus brevis DSM* 9209, *Lactobacillus plantarum DSM 9208, Leuconostoc* mesenteroides DSM 9207 et Pediococcus pentosaceus DSM 9210.

## Claims

1. Method of producing a biomass consisting of yeast and lactic acid bacteria, on a cereal medium, the product being directly utilizable as ferment for breadmaking without prior separation between the culture medium and biomass, **characterized in that** at least one yeast strain and at least one strain of lactic bacterium is cocultured, without regulation of pH, in mixed culture and/or sequential culture, on a culture medium obtained:
- by a double hydrolysis of an aqueous dilute mixture consisting of water, wholemeal flour and/or wheat germ as well as yeast autolysate and sea salt, namely
- by the complete hydrolysis of starch to fermentable sugars by the action of at least one alpha-amylase and at least one amyloglucosidase, and
- by the controlled hydrolysis of at least part of the gluten by food-grade proteolytic enzymes;
- this culture medium being free of any chemical additive other than the ingredients mentioned above.

2. Method according to Claim 1, **characterized in that** the culture is of the fed-batch type.

3. Method according to Claim 1 or 2, **characterized in that** the content of ethanol in the culture is controlled by regulating the rate of feeding the culture medium.

4. Method to one of Claims 1 to 3, **characterized in that** the partial pressure of oxygen dissolved in the culture is controlled by regulating the supply of air according to a previously defined slope maintaining the culture in an oxygen-limited metabolism.

5. Method according to one of Claims 1 to 3, **characterized in that** the partial pressure of oxygen dissolved in the culture is controlled by the rate of stirring according to a previously defined slope maintaining the culture in an oxygen-limeted metabolism.

6. Method according to one of Claims 1 to 5, **characterized in that** the yeast used is a strain of *Saccharomyces cerevisiae.*

7. Method according to Claim 6, **characterized in that** the yeast used is a strain of *Saccharomyces cerevisiae* isolated from a natural leaven.

8. Method according to Claim 7, **characterized in that** the yeast used is the *Sac charomyces cerevisiae steineri* DSM 9211 strain.

9. Method according to one of Claims 1 to 8, **characterized in that** the lactic acid bacterium used is of the genera *Lactobacillus, Leuconostoc* and/or *Pediococcus.*

10. Method according to Claim 9, **characterized in that** the lactic acid bacterium used is a strain of *Lactobacillus brevis, Lactobacillus plantarum, Leuconostoc mesenteroides* and/or *Pediococcus pentosacus.*

11. Method according to Claim 10, **characterized in that** the lactic acid bacterium used is one or more of the strains *Lactobacillus brevis* DSM 9209, *Lactobacillus plantarum* DSM 9208, *Leuconostoc mesenteroides* DSM 9207 und *Pediococcus pentosacus* DSM 9210.

12. Method according to one of Claims 1 to 11, **characterized in that** the coculture is carried out in a sequential mixed culture.

13. Method according to Claim 12, **characterized in that** the ethanol content during the method of culture varies between 0.5 and 10 gramme/litre of coculture.

14. Method according to one of Claims 1 to 13, **characterized in that**
- a basal medium is introduced into a bioreactor;
- this basal medium is inoculated with yeast and lactic acid bacteria to be cultured distributed in a proportioning medium;
- the bioreactor is continuously fed with the proportioning medium;
while maintaining a temperature of about 30°C, a controlled aeration by the partial pressure of oxygen and an ethanol concentration of between 0.5 and 10 grams/litre of culture.

15. Method according to one of Claims 1 to 14, **characterized in that** the product obtained is concentred by centrifugation or filtration.

16. Ferment for breadmaking, **characterized in that** it is the biomass obtained by the method of Claim 1 and **in that** it comprises as yeast the *Saccharomyces cerevisiae steineri* DSM 9211 strain and as lactic acid bacterium one or more of the strains *Lactobacillus brevis* DSM 9209, *Lactobacillus plantarum* DSM 9208, *Leuconostoc mesenteroides* DSM 9207 and *Pediococcus pentosacus* DSM 9210.

## Patentansprüche

1. Verfahren zur Gewinnung einer Biomasse, welche aus Hefe und Milchsäurebakterien besteht, auf einem Getreidemedium, wobei das erhaltene Produkt ohne vorgängige Trennung von Kulturmedium und Biomasse direkt als Ferment für die Brotherstellung verwendbar ist, **dadurch gekennzeichnet, dass** man ohne pH-Regulierung mindestens einen Hefestamm und mindestens einen Stamm eines Milchsäurebakteriums in Mischkultur und/oder sequentieller Kultur auf einem Medium züchtet, welches erhalten wurde:
- durch eine doppelte Hydrolyse einer verdünnten wässerigen Mischung, welche aus Wasser, Vollkornmehl und/oder Weizenkeimen sowie Hefeautolysat und Meersalz besteht, nämlich
- durch die totale Hydrolyse der Stärke zu vergärbaren Zuckern durch Einwirkung wenigstens einer alpha-Amylase und wenigstens einer Amyloglucosidase, und
- durch gesteuerte Hydrolyse wenigstens eines Teils des Glutens durch proteolytische Enzyme von Lebensmittelqualität;
wobei dieses Kulturmedium frei ist von jeglichen chemischen Zusätzen ausser den vorstehend genannten.

2. Verfahren nach Anspruch 2, **dadurch gekennzeichnet, dass** die Kultur vom Typ versorgte diskontinuierliche Kultur ist.

3. Verfahren nach Anspruch 1 oder 2, **dadurch gekennzeichnet, dass** man den Äthanolgehalt in der Kultur durch Regulierung der Versorgungsgeschwindigkeit des Kulturmediums steuert.

4. Verfahren nach einem der Ansprüche 1 bis 3, **dadurch gekennzeichnet, dass** man den Partialdruck des in der Kultur gelösten Sauerstoffs durch Regulierung der Luftzufuhr gemäss einer vorbestimmten Steigung steuert, wobei die Kultur bei einem beschränkten Sauerstoffstoffwechsel gehalten wird.

5. Verfahren nach einem der Ansprüche 1 bis 3, **dadurch gekennzeichnet, dass** man den Partialdruck des in der Kultur gelösten Sauerstoffs durch Regulierung der Rührgeschwindigkeit gemäss einer vorbestimmten Steigung steuert, wobei die Kultur bei einem beschränkten Sauerstoffstoffwechsel gehalten wird.

6. Verfahren nach einem der Ansprüche 1 bis 5, **dadurch gekennzeichnet, dass** die eingesetzte Hefe ein Stamm von *Saccharomyces cerevisiae ist.*

7. Verfahren nach Anspruch 6, **dadurch gekennzeichnet, dass** die eingesetzte Hefe ein aus einem natürlichen Hebel isolierter Stamm von *Saccharomyces cerevisiae* ist.

8. Verfahren nach Anspruch 7, **dadurch gekennzeichnet, dass** die eingesetzte Hefe der Stamm *Saccharomyces cerevisiae steineri* DSM 9211 ist.

9. Verfahren nach einem der Ansprüche 1 bis 8, **dadurch gekennzeichnet, dass** das eingesetzte Milchsäurebakterium der Gattung *Lactobacillus, Leuconostoc* und/oder *Pediococcus* angehört.

10. Verfahren nach Anspruch 9, **dadurch gekennzeichnet, dass** das eingesetzte Milchsäurebakterium ein Stamm von *Lactobacillus brevis, Lactobacillus plantarum, Leuconostoc mesenteroides* und/oder *Pediococcus pentosacus* ist.

11. Verfahren nach Anspruch 10, **dadurch gekennzeichnet, dass** das eingesetzte Milchsäurebakterium einer oder mehrere der Stämme *Lactobacillus brevis* DSM *9209, Lactobacillus plantarum* DSM 9208, *Leuconostoc mesenteroides* DSM 9207 und *Pediococcus pentosacus* DSM 9210 ist.

12. Verfahren nach einem der Ansprüche 1 bis 11, **dadurch gekennzeichnet, dass** die Kokultur als sequentielle Mischkultur durchgeführt wird.

13. Verfahren nach Anspruch 12, **dadurch gekennzeichnet, dass** der Äthanolgehalt während des Kulturverfahrens zwischen 0,5 und 10 Gramm/Liter variiert.

14. Verfahren nach einem der Ansprüche 1 bis 13, **dadurch gekennzeichnet, dass** man:
- ein Basismilieu in einen Bioreaktor einführt;
- dieses Basismilieu mit der zu kultivierenden Hefe und den zu kultivierenden Milchsäurebakterien, verteilt in einem Dosiermilieu, beimpft; und
- den Bioreaktor kontinuierlich mit dem Dosiermedium versorgt,
wobei eine Temperatur von etwa 30 °C, über den Sauerstoffpartialdruck eine gesteuerte Luftzufuhr sowie eine Äthanolkonzentration zwischen 0,5 und 10 Gramm/Liter aufrechterhalten wird.

15. Verfahren nach einem der Ansprüche 1 bis 14, **dadurch gekennzeichnet, dass** man das erhaltene Produkt durch Zentrifugation oder Filtration konzentriert.

16. Ferment für die Brotherstellung, **dadurch gekennzeichnet, dass** es die nach dem Verfahren von Anspruch 1 erhaltene Biomasse ist und dass es als Hefe den Stamm *Saccharomyces cerevisiae steineri* DSM 9211 und als Milchsäurebakterium einen oder mehrere der Stämme *Lactobacillus brevis* DSM 9209, *Lactobacillus plantarum* DSM 9208, *Leuconostoc mesenteroides* DSM 9207 und *Pediococcus pentosacus* DSM 9210 enthält.
